# EUROPEAN PATENT APPLICATION

(11) **EP 2 083 003 A1**
(43) Date of publication of application: **29.07.2009**
(21) Application number: 07721593.7
(22) Date of filing: 28.06.2007
(51) Int. Cl.: C07C 17/278, C07C 19/01, B01J 27/128, B01J 31/18, B01J 31/30

(54) **PROCESS FOR PREPARATION OF 1,1,1,3,3-PENTACHLOROBUTANE**

(30) Priority: 17.11.2006 CN 200610154742
(71) Applicant: ZheJiang Lantian Environmental Protection Hi-Tech Co. Ltd., Hangzhou Zhejiang 310012 (CN)
(72) Inventor: HAN, Guoqing, Zhejiang 310023 (CN); WU, Jiangping, Zhejiang 310023 (CN); YANG, Jian, Zhejiang 310023 (CN); FANG, Xiaoqing, Zhejiang 310023 (CN)
(74) Representative: Tombling, Adrian George
(86) International application number: PCT/CN2007/002022
(87) International publication number: WO 2008/058444

(57) **Abstract**

A process for preparation of 1,1,1,3,3-pentachlorobutane comprises reacting carbon tetrachloride and 2-chloropropene, in which the catalyst includes main catalyst and co-catalyst. The said main catalyst is one or more catalysts selected from the group consisting of Fe (II) salt, Fe or Fe (III) salt, co-catalyst is one or more catalysts selected from the group consisting of alkyl phosphites, alkyl phosphates or aryl phosphates. The said process has high yield and selectivity, and the catalyst is conveniently separated from the product.

## Description

### Technical Field

This invention relates to a process for preparing 1,1,1,3,3-pentachlorobutane, and more particularly, to a process for preparing 1,1,1,3,3-pentachlorobutane from 2-chloropropylene and carbon tetrachloride.

### Background Art

Currently, a variety of processes have been developed for preparing 1,1,1,3,3-pentachlorobutane, including one process in which 2-chloropropylene and carbon tetrachloride are used as starting materials: 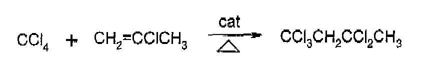 R. Freidlina et al. (Bull. Acad. Sci, 1766-1769, 1979 ), for example, have reported the preparation of 1,1,1,3,3-pentachlorobutane from 2-chloropropylene and carbon tetrachloride using Fe(CO)₅-ethanol or isopropanol as catalyst. This process, however, involves toxic catalyst and has low yield of 1,1,1,3,3-pentachlorobutane, which limit its industrial application.

The Chinese Invention Patent Application CN 1161319 (published on October 8, 1997), with the title of "1,1,1,3,3-pentachlorobutane and 1,1,1,3,3-pentafluorobutane", disclosed the preparation of 1,1,1,3,3-pentachlorobutane from carbon tetrachloride and 2-chloropropylene using copper salt, cuprous salt and amine as catalyst, in which the conversion rate of 2-chloropropylene starting material is up to 95% and the selectivity to 1,1,1,3,3-pentachlorobutane up to 90%. However, the copper salt, cuprous salt and amine used as catalyst in this process may result in the formation of a viscous semi-solid after the reaction. The formed semi-solid is difficult to separate in actual operation unless being washed with water, which in turn involves the treatment of waste water and the recycling of copper ion, and thus increases the cost.

The Chinese Invention Patent Application CN 1384812A (published on December 11, 2002) disclosed a continuous process for preparing halo-substituted compounds, in which the reaction between a haloalkane and a haloalkene is catalyzed by a catalyst complex with a boiling point higher than that of the reaction product. More particularly, the catalyst complex is formed from a metallic catalyst and an organic ligand, in which the metallic catalyst refers to elementary powders, salts and organometallic compounds of transition metals; the metallic catalyst preferably comprises copper and iron; and suitable organic ligands comprise amines, nitriles, amides and phosphate esters. However, the application has not shown the effect of the catalyst complex by particular reactions.

Thus, it is still desirable to further improve the process for preparing 1,1,1,3,3-pentachlorobutane from 2-chloropropylene and carbon tetrachloride.

### Contents of the Invention

The object of the invention is to provide a process for preparing 1,1,1,3,3-pentachlorobutane, comprising reacting 2-chloropropylene with carbon tetrachloride in the presence of a catalyst, wherein the catalyst comprises a main catalyst and an auxiliary catalyst, said main catalyst being one or more members selected from the group of ferrous (II) salt, iron and ferric (III) salt and said auxiliary catalyst being one or more members selected from the group of alkyl phosphite, alkyl phosphate and aryl phosphate.

The invention also provides use of a catalyst in the preparation of 1,1,1,3,3-pentachlorobutane from 2-chloropropylene and carbon tetrachloride, said catalyst comprising a main catalyst and an auxiliary catalyst, in which the main catalyst is one or more members selected from the group of ferrous (II) salt, iron and ferric (III) salt and the auxiliary catalyst is one or more members selected from the group of alkyl phosphite, alkyl phosphate and aryl phosphate.

### Mode of carrying out the invention

The inventive process for preparing 1,1,1,3,3-pentachlorobutane comprises reacting 2-chloropropylene with carbon tetrachloride in the presence of a catalyst, wherein the catalyst comprises a main catalyst and an auxiliary catalyst, said main catalyst being one or more members selected from the group of ferrous (II) salt, iron and ferric (III) salt and said auxiliary catalyst being one or more members selected from the group of alkyl phosphite, alkyl phosphate and aryl phosphate.

In the process, the 2-chloropropylene and carbon tetrachloride starting materials are known.

In the catalyst used in the process, the main catalyst is one or more members selected from the group of ferrous salt, iron and ferric salt. That is to say, either one of ferrous salt, iron and ferric salt alone or a mixture thereof can be used. When used as mixture, the members selected from the group of ferrous salt, iron and ferric salt can be present in any ratio. There is no specific limitation to the forms of ferrous salt, iron and ferric salt.

Preferably, the ferrous salt and the ferric salt are a halide.

More preferably, the ferrous salt is ferrous chloride, and the ferric salt is ferric chloride or ferric bromide.

The iron is preferably fine iron powders. More preferably, the iron has the particle size of 20-300 mesh.

In the catalyst used in the process, the auxiliary catalyst is one or more members selected from the group of alkyl phosphite, alkyl phosphate and aryl phosphate. That is to say, either one of alkyl phosphite, alkyl phosphate and aryl phosphate alone or a mixture thereof can be used. When used as mixture, the members selected from the group of alkyl phosphite, alkyl phosphate and aryl phosphate can be present in any ratio.

The alkyl phosphite is preferably C₁₋₈-alkyl phosphite, more preferably C₁₋₄-alkyl phosphite; it is further preferably tri(C₁₋₈-alkyl) phosphite, more preferably tri(C₁₋₄-alkyl) phosphite, and especially triethyl phosphite.

The alkyl phosphate is preferably C₁₋₈-alkyl phosphate, more preferably C₁₋₄-alkyl phosphate; it is further preferably tri(C₁₋₈-alkyl) phosphate, more preferably tri(C₁₋₄-alkyl) phosphate, and especially trimethyl phosphate, triethyl phosphate or tributyl phosphate.

The aryl phosphate is preferably C₆₋₁₀-aryl phosphate; it is further preferably tri(C₆₋₁₀-aryl) phosphate, more preferably triphenyl phosphate.

In a preferred catalyst, the main catalyst is one or more members selected from the group of iron and ferric salt, and the auxiliary catalyst is one or more members selected from the group of alkyl phosphite, alkyl phosphate and aryl phosphate.

In another preferred catalyst, the main catalyst is one or more members selected from the group of ferrous salt, iron and ferric salt, and the auxiliary catalyst is one or more members selected from the group of alkyl phosphate and aryl phosphate.

In one particularly preferred embodiment of the inventive process, the catalyst does not only consist of ferrous salt and alkyl phosphite. In other words, in this embodiment, the catalyst can comprise one or more members selected from the group of ferrous salt, iron and ferric salt and one or more members selected from the group of alkyl phosphite, alkyl phosphate and aryl phosphate, exclusive of the catalyst consisting of only ferrous salt and only alkyl phosphite.

The catalyst used in the invention can be prepared by mixing one or more members selected from the group of ferrous salt, iron and ferric salt and one or more members selected from the group of alkyl phosphite, alkyl phosphate and aryl phosphate, if necessary with stirring.

The catalyst used in the invention can be used directly after being prepared, or by mixing one or more members selected from the group of ferrous salt, iron and ferric salt and one or more members selected from the group of alkyl phosphite, alkyl phosphate and aryl phosphate in the starting materials and then heating the resulting mixture to reaction temperature. However, it is preferred to prepare the catalyst first and then apply it in the reaction system.

In the inventive process, the molar ratio of the main catalyst to the carbon tetrachloride starting material is in the range of 0.005-0.5 : 1, preferably in the range of 0.01-0.1 : 1.

In the inventive process, the molar ratio of the auxiliary catalyst to the carbon tetrachloride starting material is in the range of 0.005-0.5 : 1, preferably in the range of 0.01-0.1 : 1.

In the inventive process, the molar ratio of the starting material carbon tetrachloride to 2-chloropropylene is generally in the range of 0.5-10 : 1, preferably in the range of 1.5-4 : 1.

The reaction temperature in the inventive process is in the range of 50°C-130°C, preferably in the range of 70 °C-120°C, particularly preferably in the range of 75°C-105°C.

The inventive process is carried out in the liquid phase, and the reaction system can be pressurized if necessary to keep the starting materials in the liquid phase. In this regard, the desired pressure range can be easily determined by those skilled in the art.

The reaction time in the inventive process, depending on various parameters such as the reaction temperature, the concentration of the catalyst and the molar ratio of the reactants, can be determined by those skilled in the art based on particular conditions. However, it is preferred that the reaction time is 0.5-15 hours, preferably 3-12 hours, more preferably 4.5-10.5 hours.

The inventive process can be carried out in continuous, semi-continuous or batchwise mode. If in continuous mode, 2-chloropropylene, carbon tetrachloride and the catalyst can be fed into a reactor continuously. In semi-continuous or batchwise mode, carbon tetrachloride and the catalyst, for example, can be fed first in a reactor, and then 2-chloropropylene can be fed continuously or in batch.

After the reaction has completed, the reaction mixture can be worked up by filtration or precipitation to separate a solid phase containing the catalyst and an organic phase. The organic phase can be further separated by distillation or chromatography to obtain the starting materials and the product. The unreacted starting materials and the recovered catalyst can be recycled.

The inventive process allows the easy separation of the catalyst and the desired product, and also has high conversion rate of 2-chloropropylene and high selectivity to 1,1,1,3,3-pentachlorobutane.

Thus, the invention also provides use of a catalyst in the preparation of 1,1,1,3,3-pentachlorobutane from 2-chloropropylene and carbon tetrachloride, said catalyst comprising a main catalyst and an auxiliary catalyst, in which the main catalyst is one or more members selected from the group of ferrous (II) salt, iron and ferric (III) salt and the auxiliary catalyst is one or more members selected from the group of alkyl phosphite, alkyl phosphate and aryl phosphate. All the foregoing descriptions concerning ferrous salt, iron and ferric salt, and alkyl phosphite, alkyl phosphate and aryl phosphate apply to this use.

The invention will be described by the following examples. The examples are given for the purpose of illustration without any limitations to the invention.

In each example, an airtight system was used unless indicated otherwise. All the main catalyst, auxiliary catalyst and carbon tetrachloride were initially charged in a reactor, and then 2-chloropropylene was fed continuously. The pressure was not controlled during the reaction process.

In each example, the iron as main catalyst was in the form of particles of 50-200 mesh, unless indicated otherwise.

In each example, the reaction mixture was subjected to precipitation after the reaction, and the thus-obtained liquid phase was analyzed by gas chromatography to determine the conversion rate of 2-chloropropylene and the selectivity to 1,1,1,3,3-pentachlorobutane, in which:

Conversion rate of 2-chloropropylene = (the molar amount of 2-chloropropylene starting material - the molar amount of unreacted 2-chloropropylene)/ the molar amount of 2-chloropropylene starting material × 100%;

Selectivity to 1,1,1,3,3-pentachlorobutane = the molar amount of 1,1,1,3,3-pentachlorobutane produced/(the molar amount of 2-chloropropylene starting material - the molar amount of unreacted 2-chloropropylene) × 100%.

### Example 1

A reaction was conducted at a temperature of 95°C using ferrous chloride as main catalyst and triethyl phosphite as auxiliary catalyst. The molar ratio of the materials used was carbon tetrachloride: 2-chloropropylene: ferrous chloride: triethyl phosphite = 1: 0.4: 0.02: 0.02, with a total amount of 1.44 mol. The reaction was lasted for 5 hours under the pressure of no more than 0.45 MPa. After the end of the reaction, the conversion rate of 2-chloropropylene was determined to be 98% and the selectivity to 1,1,1,3,3-pentachlorobutane based on 2-chloropropylene was 90%.

### Example 2

A reaction was conducted at a temperature of 85°C using iron and ferric chloride as main catalysts and trimethyl phosphate as auxiliary catalyst. The molar ratio of the materials used was carbon tetrachloride: 2-chloropropylene; iron and ferric chloride: trimethyl phosphate = 1: 0.4; 0.03: 0.02, with a total amount of 1.45 mol. The main catalysts and the auxiliary catalyst were first mixed and charged into a reactor, and then carbon tetrachloride was added. The reaction was lasted for 6 hours under the pressure of no more than 0.40 MPa. After the end of the reaction, the conversion rate of 2-chloropropylene was determined to be 98% and the selectivity to 1,1,1,3,3-pentachlorobutane based on 2-chloropropylene was 93%.

### Example 3

A reaction was conducted at a temperature of 85°C using iron and ferric chloride as main catalysts and mixed esters of triethyl phosphate and tributyl phosphate as auxiliary catalysts. The molar ratio of the materials used was carbon tetrachloride: 2-chloropropylene: iron and ferric chloride: the mixed phosphate esters = 1_{:} 0.4: 0.03: 0.02, with a total amount 1.45 mol. The reaction was lasted for 6 hours under the pressure of no more than 0.35 MPa. After the end of the reaction, the conversion rate of 2-chloropropylene was determined to be 99% and the selectivity to 1,1,1,3,3-pentachlorobutane based on 2-chloropropylene was 95%.

### Example 4

A reaction was conducted at a temperature of 85°C using iron and ferric chloride as main catalysts and mixed esters of triethyl phosphite and triethyl phosphate as auxiliary catalysts. The molar ratio of the materials used was carbon tetrachloride: 2-chloropropylene: iron and ferric chloride: the mixed esters = 1: 0.4: 0.03: 0.02, with a total amount of 1.45 mol. The reaction was lasted for totally 6 hours under the pressure of no more than 0.45 MPa. 2-chloropropylene was charged into the reaction system in three portions at equal amounts at the beginning of the reaction, after the reaction was conducted for 2 hours and for 4 hours, respectively. After the end of the reaction, the conversion rate of 2-chloropropylene was determined to be 99% and the selectivity to 1,1,1,3,3-pentachlorobutane based on 2-chloropropylene was 95%.

### Example 5

A reaction was conducted at a temperature of 80°C using ferrous chloride as main catalyst and tributyl phosphate as auxiliary catalyst. The molar ratio of the materials used was carbon tetrachloride: 2-chloropropylene: ferrous chloride: tributyl phosphate = 1: 0.4: 0.035: 0.025, with a total amount of 1.46 mol. The reaction was lasted for 6 hours under the pressure of no more than 0.35 MPa. After the end of the reaction, the conversion rate of 2-chloropropylene was determined to be 98% and the selectivity to 1,1,1,3,3-pentachlorobutane based on 2-chloropropylene was 96%.

### Example 6

A reaction was conducted at a temperature of 80°C using the solid recovered from examples 2-4 as main catalyst and tributyl phosphate as auxiliary catalyst. The molar ratio of the materials used was carbon tetrachloride: 2-chloropropylene: recovered iron salts: tributyl phosphate = 1: 0.4: 0.025: 0.025, with a total amount of 1.45 mol. The reaction was lasted for 8 hours under the pressure of no more than 0.35 MPa. After the end of the reaction, the conversion rate of 2-chloropropylene was determined to be 98% and the selectivity to 1,1,1,3,3-pentachlorobutane based on 2-chloropropylene was 95%.

### Example 7

A reaction was conducted at a temperature of 90°C using ferric bromide as main catalyst and tributyl phosphate and triphenyl phosphate as auxiliary catalysts. The molar ratio of the materials used was carbon tetrachloride: 2-chloropropylene: ferric bromide: the mixed phosphate esters = 1: 0.5: 0.01: 0.01, with a total amount of 1.52 mol. The reaction was lasted for 5 hours. The pressure during the telomeric reaction was in the range of 0.3 MPa - 0.5 MPa. After the end of the reaction, the conversion rate of 2-chloropropylene was determined to be 97.6% and the selectivity to 1,1,1,3,3-pentachlorobutane based on 2-chloropropylene was 95%.

### Example 8

A reaction was conducted at a temperature of 100 °C using iron and ferrous chloride as main catalysts and tributyl phosphite as auxiliary catalyst, in which the iron used was fine powders of pure iron metal with the particle size of 100 mesh. The molar ratio of the materials used was carbon tetrachloride: 2-chloropropylene: iron and ferrous chloride: tributyl phosphite = 1: 0.6: 0.01: 0.01, with a total amount of 1.62 mol. The reaction was lasted for 9 hours under the pressure of no more than 0.5 MPa. After the end of the reaction, the conversion rate of 2-chloropropylene was determined to be 98.5% and the selectivity to 1,1,1,3,3-pentachlorobutane based on 2-chloropropylene was 95%.

### Example 9

A reaction was conducted at a temperature of 100 °C using a mixture of iron, ferrous chloride and ferric chloride as main catalyst and diethyl phosphite as auxiliary catalyst, in which the iron used was fine powders of pure iron metal with the particle size of 80 mesh. The molar ratio of the materials used was carbon tetrachloride: 2-chloropropylene: iron, ferrous chloride and ferric chloride: diethyl phosphite = 1: 0.5: 0.01: 0.02, with a total amount of 1.53 mol. The reaction was lasted for 8 hours under the pressure of no more than 0.5 MPa. After the end of the reaction, the conversion rate of 2-chloropropylene was determined to be 99% and the selectivity to 1,1,1,3,3-pentachlorobutane based on 2-chloropropylene was 95%.

### Example 10

A reaction was conducted at a temperature of 90°C using a mixture of ferric bromide and ferric chloride as main catalyst and triphenyl phosphite as auxiliary catalyst. The molar ratio of the materials used was carbon tetrachloride: 2-chloropropylene: ferric bromide and ferric chloride: triphenyl phosphite = 1: 0.5: 0.04; 0.01, with a total amount of 1.55 mol. The reaction was lasted for 8 hours under the pressure of no more than 0.3 MPa. After the end of the reaction, the conversion rate of 2-chloropropylene was determined to be 98% and the selectivity to 1,1,1,3,3-pentachlorobutane based on 2-chloropropylene was 95.5%.

## Claims

1. A process for preparing 1,1,1,3,3-pentachlorobutane, comprising reacting 2-chloropropylene with carbon tetrachloride in the presence of a catalyst, wherein the catalyst comprises a main catalyst and an auxiliary catalyst, said main catalyst being one or more members selected from the group of ferrous (II) salt, iron and ferric (III) salt and said auxiliary catalyst being one or more members selected from the group of alkyl phosphite, alkyl phosphate and aryl phosphate.

2. The process of claim 1, **characterized in that** the ferrous (II) salt and the ferric (III) salt as main catalysts are a halide, preferably ferrous chloride, ferric chloride or ferric bromide.

3. The process of claim 1, **characterized in that** the iron is fine powders of pure iron metal with the particle size of 20-300 mesh.

4. The process of claim 1, **characterized in that** the alkyl phosphite is C₁₋₈-alkyl phosphite, preferably C₁₋₄-alkyl phosphite, and more preferably triethyl phosphite.

5. The process of claim 1, **characterized in that** the alkyl phosphate is tri(C₁₋₈-alkyl) phosphate, preferably tri(C₁₋₄-alkyl) phosphate, and more preferably trimethyl phosphate, triethyl phosphate or tributyl phosphate.

6. The process of claim 1, **characterized in that** the aryl phosphate is triphenyl phosphate.

7. The process of claim 1, **characterized in that** the catalyst does not only consist of ferrous (II) salt and alkyl phosphite.

8. The process of any of claims 1-7, **characterized in that** the molar ratio of the catalyst to carbon tetrachloride is:
the main catalyst: carbon tetrachloride in the range of 0.005-0.5 : 1, preferably 0.01-0.1 : 1; and
the auxiliary catalyst: carbon tetrachloride in the range of 0.005-0.5 : 1, preferably 0.01-0.1 : 1.

9. The process of any of claims 1-7, **characterized in that** the reaction temperature is in the range of 50 °C -130 °C, preferably in the range of 70 °C -120 °C, and more preferably in the range of 75°C-105°C.

10. Use of a catalyst in the preparation of 1,1,1,3,3-pentachlorobutane from 2-chloropropylene and carbon tetrachloride, said catalyst comprising a main catalyst and an auxiliary catalyst, in which the main catalyst is one or more members selected from the group of ferrous (II) salt, iron and ferric (III) salt, and the auxiliary catalyst is one or more members selected from the group of alkyl phosphite, alkyl phosphate and aryl phosphate.
